(19) Europäisches Patentamt
European Patent Office
Office européen des brevets

(11) Numéro de publication: **0 396 447 A1**

(12) **DEMANDE DE BREVET EUROPEEN**

(21) Numéro de dépôt: **90401068.3**

(51) Int. Cl.[5]: **C12P 7/62, C12P 41/00**

(22) Date de dépôt: **20.04.90**

(30) Priorité: **02.05.89 FR 8905808**

(43) Date de publication de la demande: **07.11.90 Bulletin 90/45**

(84) Etats contractants désignés: **AT BE CH DE DK ES FR GB GR IT LI LU NL SE**

(71) Demandeur: **RHONE POULENC CHIMIE**
**25 Quai Paul Doumer**
**F-92408 Courbevoie Cédex(FR)**

(72) Inventeur: **Aviron-Violet, Paul**
**25bis, Chemin de la Citadelle**
**F-69230 Saint Genis Laval(FR)**

(74) Mandataire: **Ricalens, François**
**RHONE-POULENC CHIMIE Service Brevets**
**Chimie 25, quai Paul Doumer**
**F-92408 Courbevoie(FR)**

(54) **Procédé de séparation énantiomériquement sélective des esters des acides halogéno-2 propioniques.**

(57) La présente invention concerne un procédé de séparation des deux isomères optiques des acides halogéno-2 propioniques.

Ce procédé consiste à réaliser une transestérification sélective d'un ester d'un des deux isomères de l'acide halogéno-2 propionique à l'aide d'une enzyme.

EP 0 396 447 A1

# Procédé de séparation énantiomériquement sélective des esters des acides halogéno-2 propioniques

La présente invention concerne un procédé de séparation énantiomériquement sélective des esters des acides halogéno-2 propioniques. Elle concerne plus particulièrement un procédé de séparation enzymatique par transestérification d'un des deux isomères optiques des chloro-2 propioniates d'alkyle.

Les esters optiquement purs de l'acide chloropropionique sont utilisés dans les domaines pharmaceutiques et agrochimiques. Les produits finaux médicaments, herbicides, fongicides .. sont souvent actifs que sous une seule forme énantiomère, la forme opposée n'ayant soit pas d'activité, soit même pouvant présenter une certaine toxicité.

Pour des molécules aussi simples chimiquement que les esters de l'acide chloropropionique, l'industrie chimique cherche depuis longtemps le moyen de séparer ou de synthétiser majoritairement un isomère au détriment de l'autre.

La plus ancienne voie de synthèse décrite dans le brevet FR2459221, de l'ester de l'acide chloropropionique consiste à partir de l'acide lactique (R), synthétisé de façon optiquement pure par voie microbiologique, à l'esterifier puis à faire réagir sur l'ester de l'acide (R) lactique, le chlorure de thionyle.

Ce procédé permet d'obtenir un ester de l'acide chloropropionique optiquement pur par une voie qui économiquement n'est pas la plus favorable. En effet, la synthèse de l'acide lactique optiquement pur est onéreuse, son estérification et sa chloration avec des exigences de maintien de la pureté optique sont aussi coûteuses. Par contre, l'acide chloropropionique et ses esters sous forme racémique sont disponibles sur le marché des produits chimiques à un prix très avantageux.

Aussi l'industrie cherche depuis de nombreuses années une méthode permettant de dédoubler l'acide chloropropionique ou ses dérivés de façon concurrentielle par rapport à l'ancien procédé à partir de l'acide lactique optiquement pur.

Certaines méthodes de dédoublement des acides halogénoropioniques ou de ses dérivés racémiques ont déjà été décrites. Les méthodes stéréosélectives peuvent être divisées en trois groupes : les méthodes d'estérification enzymatique, les méthodes d'hydrolyse enzymatique, les méthodes d'hydrolyse-deshalogénation enzymatique.

Parmi les méthodes d'estérification enzymatique stéréosélectives, on peut citer le brevet US 4 601 987 qui décrit l'estérification par un alcool contenant 1 à 4 atomes de carbone, au moyen d'une lipase de Candida cylindracea des acides halogénopropioniques. Cette lipase permet d'obtenir spécifiquement l'isomère (R) de l'ester recherché laissant l'acide propionique (S). La séparation de l'ester et de l'acide n'est pas toujours facile.

L'acide (S) propionique est séparé de l'ester indésiré par extraction à l'eau après neutralisation, l'ester restant dans la phase organique. Celui-ci est alors hydrolysé en milieu acide puis racémisé avec un acide dont l'hydrogène correspond à celui porté par l'acide propionique puis réintroduit dans la phase d'estérification stéréosélective. L'ester est dans cette étape, parce que la lipase est sélective de l'isomère (R), toujours le composé non désiré, il doit donc être hydrolysé, racémisé puis recyclé. Il serait plus avantageux d'obtenir directement l'ester (S) et de recycler l'acide (R) mais il n'est pas possible d'inverser le sens du travail d'une enzyme.

L'acide S correspondant au résidu après estérification, ne peut pas être obtenu pur si l'estérification n'est pas complète. Or, il est difficile dans une voie enzymatique d'obtenir un taux de transformation de 100 %. Cette voie ne permet pas d'atteindre le dérivé désiré de manière sélective.

Ce procédé n'est donc pas économiquement concurrentiel de l'ancien procédé par voie chimique évoqué précédemment.

Parmi les méthodes d'hydrolyse enzymatique, on peut citer le brevet EP196625 qui décrit l'hydrolyse stéréosélective des esters halogénopropioniques racémiques au moyen d'une lipase provenant de Candida cylindracea permettant d'obtenir spécifiquement l'isomère (R) de l'acide halogénopropionique tout en conservant l'ester (S) de l'acide halogénopropionique.

L'hydrolyse n'étant jamais complète (TT<100 %), il subsiste toujours au sein de l'isomère (S) de l'ester un peu de composé de départ racémique qu'il n'est pas possible de séparer. Le problème est le même que dans le cas précédent.

L'industrie cherche donc un procédé permettant de synthétiser l'ester (S) de l'acide halogénopropionique à partir d'acide ou d'un dérivé de l'acide halogénopropionique en laissant dans le milieu réactionnel le mélange racémique.

Parmi le troisième groupe de méthode permettant de dédoubler les acides halogénopropioniques, on peut citer le brevet EP 179603 qui consiste à hydrolyser sélectivement l'halogène porté par l'acide propionique. Cette hydrolyse sélective est réalisée au moyen d'une halidohydrolase. Au moins une partie

de l'isomère (R) est transformée avec inversion de configuration en acide (S) hydroxypropionique encore appelée acide (S) lactique. L'acide (S) halogénopropionique subsiste dans le milieu réactionnel et le taux de transformation de l'isomère (A) n'étant pas complet comme dans les deux brevets précédemment cités, il est possible par cette technique d'obtenir l'acide (S) halogénopropionique de manière pure.

La présente invention a permis de résoudre les problèmes laissés non résolus par l'art antérieur, c'est-à-dire à partir d'un mélange racémique d'ester des acides halogénopropioniques, obtenir de manière stéréospécifique un seul des 2 isomères des esters de l'acide halogénopropionique racémique de départ.

Cette résolution optique a pu être atteinte par une réaction de transestérification réalisée de manière spécifique sur l'isomère (S) de l'ester halogénopropionique.

L'ester de l'acide halogénopropionique de départ est un ester d'alkyle dont le groupe alkyle contient 1 à 12 atomes de carbone.

On préfère utiliser un ester d'alkyle contenant 1 à 4 atomes de carbone et tout particulièrement on préfère partir de l'ester méthylique ou éthylique d'un acide halogénopropionique.

L'ester de l'acide halogénopropionique est notamment un ester de l'acide chloro ou bromopropionique.

La réaction peut être schématisée de la façon suivante :

$$\underset{(S,R)}{CH3 - \overset{\overset{X}{|}}{C}H - COOR1} + R2\ OH \rightarrow \underset{(S)}{CH3 - \overset{\overset{X}{|}}{C}H - COOR2} + \underset{(R)}{CH_3 - \overset{\overset{X}{|}}{C}H - COOR_1}$$

dans laquelle R1 et R2 sont de préférence des groupes alkyle contenant 1 à 4 atomes de carbone et tout particulièrement R1 est un groupe méthyle ou éthyle et R2 est un groupe isobutyle.

L'isomère (S) recherché même si la réaction n'est pas quantitative (taux de transformation < 100 %) sera facilement séparé par distillation, l'ester isobutylique ayant un point d'ébullition différent de celui de l'ester méthylique ou éthylique. Le milieu réactionnel résiduel contient l'ester isobutylique (S) optiquement pur. Le distillat est racémisé après hydrolyse par un acide fort.

La transestérification est réalisée au moyen d'une poudre enzymatique provenant de la culture d'un microorganisme choisi parmi Penicillium cyclopium ou Geotricum candidum .

On préfère utiliser la souche de Geotricum candidum déposée au "Centraalbureau voor Schimmelcultures", sous le n° 100-89.

Cette souche est de préférence cultivée sur un milieu de fermentation classique contenant en plus une huile végétale et de préférence l'huile de soja.

La culture de Geotrichum candidum, souche CBS 100-89 peut être effectuée par toute méthode de culture aérobie en surface ou en profondeur, mais cette dernière est à préférer pour des raisons de commodité. On utilise à cette fin les techniques d'ensemencement et de fermentation et les différents types d'appareils qui sont d'un usage courant dans l'industrie des fermentations.

Le milieu de fermentation contient de manière classique essentiellement des sources de carbone et d'azote assimilables, des éléments minéraux et éventuellement des facteurs de croissance, tous ces éléments pouvant être apportés sous forme de produits bien définis ou par des mélanges complexes tels qu'on en rencontre dans les produits biologiques d'origines diverses.

Comme source de carbone assimilable, on peut utiliser des hydrates de carbone tels que le glucose et/ou des glycérides tels que l'huile de soja.

Les sources convenables d'azote assimilable sont extrêmement variées. Elles peuvent être des substances chimiques très simples comme les sels minéraux d'ammonium. Elles peuvent être aussi apportées par des substances complexes contenant principalement l'azote sous forme protidique telles que le corn-steep.

Parmi les éléments minéraux ajoutés, certains peuvent avoir un effet tampon ou neutralisant comme le carbonate de calcium. D'autres apportent l'équilibre ionique nécessaire au développement de Geotrichum candidum, souche CBS 100-89 comme le chlorure de cobalt.

L'enzyme peut être utilisée sous forme libre ou immobilisée. On utilise environ 0,1 à 100% en poids d'enzyme par rapport à l'ester de départ.

La réaction a lieu dans l'alcool de transestérification ou dans un solvant inerte vis-à-vis de l'alcool, de l'enzyme et de l'ester de l'acide halogénopropionique. On préfère utiliser parmi ces solvants un solvant non miscible à l'eau choisi parmi les hydrocarbures, aliphatiques ou aromatiques, les hydrocarbures aliphatiques ou aromatiques halogénés.

La concentration de l'ester calculée par rapport à l'alcool est comprise entre 0,1 et 5 moles d'ester par

litre d'alcool ou de solvant. Pour une meilleure mise en oeuvre de l'invention, la température réactionnelle est comprise entre 5 et 60° C, et de préférence entre 30 et 60° C.

La présente invention sera plus complètement décrite à l'aide de l'exemple suivant qui ne peut être considéré comme limitatif de l'invention.

## Exemple

### A - Fermentation -

On charge dans un fermenteur de 170 litres :

| | |
|---|---|
| - corn-steep (50% d'extrait sec) | 4800 g |
| - glucose monohydraté | 2400 g |
| - chlorure de sodium | 600 g |
| - sulfate de magnésium heptahydraté | 120 g |
| - eau de ville q.s.p..... | 110 litres |

Après avoir ajusté le pH à 7,50 avec 550 cm3 de soude 10N, on ajoute :
- carbonate de calcium 600 g.

Le pH du milieu est alors égal à 7,55; on stérilise le milieu par barbotage de vapeur à 122° C pendant 40 mn; après refroidissement, du fait de la condensation de la vapeur au cours de la stérilisation, le volume du bouillon est de 120 litres, le pH est égal à 6,96.

On ensemence alors avec 200 cm3 d'une culture en erlenmeyer agité de Geotrichum candidum souche CBS 100-89. La culture est développée à 28° C pendant 12 heures en agitant et en aérant avec de l'air stérile; elle est alors convenable pour l'ensemencement de la culture productrice.

La culture productrice est effectuée dans un fermenteur de 800 litres chargé avec les substances suivantes :

| | |
|---|---|
| - corn-steep (50% d'extrait sec) | 7 kg |
| - huile de soja | 7 litres |
| - sulfate d'ammonium | 0,70 kg |
| - solution de chlorure de cobalt hexahydraté, à 20 g/l | 0,35 litre |
| - eau de ville q.s.p. | 310 litres. |

Après avoir ajusté le pH à 6,40 avec 600 cm3 de soude 10N, on ajoute :
- carbonate de calcium 1,75 kg.

Le pH du milieu est alors égal à 6,60; on stérilise le milieu par barbotage de vapeur à 122° C pendant 40 mn. Après refroidissement, du fait de la condensation de la vapeur au cours de la stérilisation, le volume du bouillon est de 340 litres, il est complété à 350 litres par addition de 10 litres de solution aqueuse stérile contenant :
- glucose monohydraté 3,5 kg.

Le pH du milieu est égal à 6,75. On ensemence alors avec 3,5 litres de la culture inoculum en fermenteur de 170 litres décrite ci-dessus. La culture est développée à 28° C durant 39 heures en agitant avec une turbine tournant à 200 tours/mn et en aérant avec un volume d'air stérile de 20 $m^3$/h. Le pH du moût en fin de culture est limité à 7,00 par addition d'environ 0,5 litre d'acide chlorhydrique 6N au cours de la culture.

En fin d'opération, le volume du moût est 370 litres et l'activité lipasique du filtrat est de 10,0 U.I./cm3 (1 U.I. est la quantité d'enzyme qui libère 1 micro équivalent d'acide par minute à 25° C (Desnuelle, Constantin Baldy, Bull. Soc. Chim. Biol. 37, 285 (1955).

### B - Extraction -

370 litres de moût, obtenus ccmme décrit ci-avant et titrant 10,8 U.I./cm3, sont envoyés dans une cuve munie d'un agitateur. On ajoute 25 kg d'adjuvant de filtration (silice), on agite pendant 30 minutes puis on filtre la suspension sur filtre-presse. Le gâteau est lavé par 80 litres d'eau. Le filtrat, dont le volume total est de 380 litres et le titre enzymatique de 10,5 U.I./cm3, est concentré sous pression réduite à une température de 35° C. On obtient 60 litres de concentrat titrant 39 U.I./cm3.

On ajoute dans ce concentrat 30 kg de sulfate d'ammonium tout en maintenant le pH à 8 par addition d'ammoniaque 10N et, après 30 minutes d'agitation, on laisse reposer une nuit à 4° C. On ajoute alors 500 g d'adjuvant de filtration et on filtre. Le précipité actif et l'adjuvant de filtration ainsi isolés sont remis en suspension dans 5 litres d'eau distillée à 4° C; on agite 15 minutes en maintenant le pH à 8. La suspension est filtrée, le gâteau de filtration est lavé par 2 fois 1500 cm3 d'eau distillée froide. On obtient ainsi 0,1 litre de filtrat titrant 94 U.I./cm3.

Le filtrat est dialysé à 4° C, pendant 48 heures, à travers une membrane en cellulose régénérée, contre 50 litres d'eau distillée.

Dans le dialysat obtenu (11 litres titrant 57,5 U.I./cm3) maintenu à 4° C et à pH 8, on ajoute sous agitation 4,4 litres d'acétone préalablement refroidie à -20° C, puis on agite pendant encore 5 minutes. Le précipité actif est isolé par centrifugation à -20° C pendant 5 minutes, puis remis en suspension dans 250 cm3 d'eau distillée à 4° C. La suspension est clarifiée par une nouvelle centrifugation à 4° C pendant 10 minutes . L'insoluble est éliminé, l'acétone résiduelle est évaporée sous pression réduite (2 mm Hg) à 20° C et la solution finale est lyophilisée

On obtient 22,9 g d'enzyme titrant 18 000 U.I./g.

A 7 ml d'une solution 2 molaires de (R,S) chloropropionate d'éthyle dans de l'isobutanol sont ajoutés 330 mg d'enzyme de Geotricum candidum préparé précédemment. Le mélange est agité pendant 47 heures à 37° C Après élimination de l'enzyme par centrifugation le mélange est distillé et analysé par chromatographie en phase gazeuse, le taux de chloropropionate d'éthyle transestérifié est de 10 %, l'excès énantiomérique est déterminé par chromatographie sur une phase chirale la pureté optique de l'ester isobutylique formé est de 68 % en isomère de configuration S.

La pureté optique est égale à :

$$\frac{\text{Concentration en S - concentration en R}}{\text{Concentration en S + concentration en R}} \times 100$$

## Revendications

1. Procédé de séparation de deux isomères optiques d'un ester halogénopropionique caractérisé ce qu'on effectue une transestérification enzymatique énantiomériquement sélective et qu'on sépare l'ester optiquement pur recherché.

2. Procédé selon la revendication 1 caractérisé en ce que la transestérification est stéréosélective de l'isomère S.

3. Procédé selon la revendication 1 caractérisé en ce que l'ester halogénopropionique est un ester de l'acide chloro ou bromopropionique.

4. Procédé selon la revendication 1 caractérisé en ce que l'ester halogénopropionique est un ester de l'acide halogénopropionique et d'un alcool contenant 1 à 12 atomes de carbone et de préference 1 à 4 atomes de carbone.

5. procédé selon la revendication 4 caractérisé en ce que l'ester halogénopropionique est l'ester éthylique de l'acide chloropropionique.

6. Procédé selon la revendication 1 caractérisé en ce que la transestérification est effectuée au moyen d'un alcool contenant 1 à 12 atomes de carbone et de préférence 1 à 4 atomes de carbone.

7. Procédé selon la revendication 1 caractérisé en ce que la transestérification est effectuée au moyen de l'alcool isobutylique, de l'alcool méthylique ou de l'alcool éthylique.

8. procédé selon la revendication 1 caractérisé en ce que la transestérification est effectuée entre l'ester éthylique de l'acide chloropropionique et l'alcool isobutylique.

9. Procédé selon la revendication 1 caractérisé en ce que la transestérification enzymatique est effectuée de manière énantiomériquement sélective au moyen d'une lipase provenant de Penicillium cyclopium ou de Geotricum candidum.

10. Procédé selon la revendication 1 caractérisé en ce que la transestérification a lieu dans un solvant organique inerte non miscible à l'eau choisi parmi les hydrocarbures aromatiques ou aliphatiques.

11. Procédé selon la revendication 1 caractérisé en ce que l'ester non recherché est racémisé et recyclé à l'étape de transestérification.

Office européen des brevets

# RAPPORT DE RECHERCHE EUROPEENNE

Numero de la demande

EP 90 40 1068

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
| --- | --- | --- | --- |
| A | BIOTECHNOLOGY AND BIOENGINEERING, vol. XXVI, 1984, pages 1449-1454, John Wiley & Sons, Inc.; B. CAMBOU et al.: "Comparison of different strategies for the lipase-catalyzed preparative resolution of racemic acids and alcohols: Asymmetric hydrolysis esterification, and transesterification" * En entier * | 1 | C 12 P 7/62<br>C 12 P 41/00 |
| A | US-A-4 601 987 (A. KLIBANOV) * Revendications * | 1 | |
| A | EP-A-0 206 436 (MASSACHUSETTS INT.) * Revendications * | 1 | |
| A | CHEMICAL ABSTRACTS, vol. 105, no. 25, décembre 1986, page 627, résumé no. 224632g, Columbus, Ohio, US; & JP-A-61 111 699 (TORAY INDUSTRIES, INC.) 29-05-1986 | 1 | |

| DOMAINES TECHNIQUES RECHERCHES (Int. Cl.5) |
| --- |
| C 12 P |

Le présent rapport a été établi pour toutes les revendications

| Lieu de la recherche | Date d'achèvement de la recherche | Examinateur |
| --- | --- | --- |
| LA HAYE | 03-08-1990 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant